# EUROPÄISCHE PATENTSCHRIFT

(11) **EP 1 758 514 B1**
(45) Veröffentlichungstag und Bekanntmachung des Hinweises auf die Patenterteilung: **10.09.2008**
(21) Anmeldenummer: 04738030.8
(22) Anmeldetag: 24.06.2004
(51) Int. Cl.: A61B 17/72

(54) **MARKNAGEL**
INTRAMEDULLARY NAIL
CLOU D'OSTEOSYNTHESE INTRAMEDULLAIRE

(43) Veröffentlichungstag der Anmeldung: 07.03.2007
(73) Patentinhaber: Synthes GmbH, 4436 Oberdorf (CH)
(72) Erfinder: ULRICH, Dieter, CH-8037 Zürich (CH); FRIGG, Robert, CH-2544 Bettlach (CH)
(74) Vertreter: Lusuardi, Werther
(86) Internationale Anmeldenummer: PCT/CH2004/000389
(87) Internationale Veröffentlichungsnummer: WO 2006/000109

(56) Entgegenhaltungen:
- WO-A-00/44946
- US-A- 4 805 607
- US-A- 4 858 602
- US-B1- 6 200 685
- US-B1- 6 261 290
- DATABASE MEDLINE [Online] US NATIONAL LIBRARY OF MEDICINE (NLM), BETHESDA, MD, US; März 1998 (1998-03), WILKEY K D ET AL: "Mechanical characteristics of eight femoral intramedullary nailing systems." XP002318947 Database accession no. NLM9553858 & JOURNAL OF ORTHOPAEDIC TRAUMA. 1998 MAR-APR, Bd. 12, Nr. 3, März 1998 (1998-03), Seiten 177-185, ISSN: 0890-5339

## Beschreibung

Die Erfindung bezieht sich auf einen Marknagel gemäss dem Oberbegriff des Patentanspruchs 1.

Aus der US-A 4,875,474 BORDER ist ein Marknagel bekannt, dessen mittlerer Abschnitt durch spanabhebende Bearbeitung eine geringere Wandstärke erhalten hat und damit auch eine nachteilige geringere Festigkeit - im Vergleich zu den proximalen und distalen Endteilen des Marknagels aufweist.

Aus der US-B1 6,261,290 FRIEDL ist ein hohler Marknagel bekannt, dessen distal gelegener Schaftteil eine geringere Wandstärke aufweist als der proximal gelegene Anschlussteil. Der Schaftteil ist somit flexibler als der Anschlussteil, doch besteht auch hier der Nachteil der geringeren Festigkeit des Schaftteils; ein Nachteil, der durch die diversen Querbohrungen im Schaftteil noch potenziert wird.

Ein lokales Härtungsverfahren ist in US 6200685 offenbart, ohne ein Hinweis darauf zu geben, dass dieses, Verfahren explicit für den Schafteil eines Nagels benutzt werden kann.

Hier will die Erfindung Abhilfe schaffen. Der Erfindung liegt die Aufgabe zugrunde, einen Marknagel zu schaffen, dessen Schaftteil im Vergleich zum Anschlussteil flexibler ist aber trotzdem eine möglichst hohe Festigkeit aufweist.

Die Erfindung löst die gestellte Aufgabe mit einem Marknagel, welcher die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank dem erfindungsgemässen Marknagel trotz einer Flexibilisierung des Schaftteils eine hohe mechanische Festigkeit erzielt wird. Durch die Flexibilität wird das Einbringen des Marknagel in den Markkanal begünstigt und die höhere Materialfestigkeit senkt das Risiko eines Nagelbruches.

Ein weiterer Vorteil ergibt sich aus dem geringeren Gesamtgewicht des Nagels durch die geringere Wandstärke (ca. 30 % geringeres Gewicht gegenüber einem Nagel mit konstanter Wandstärke). Schliesslich ergibt sich auch ein wirtschaftlicheres Herstellungsverfahren für den Marknagel, weil er gegenüber dem Stand der Technik schneller hergestellt werden kann und kein Material durch spanabhebende Bearbeitung verloren geht. Dies erreicht man einerseits dadurch, dass man von einem vorgefertigten Rohr ausgehen kann und dessen Wandstärke noch verändert werden kann, und andererseits dadurch, dass das Verfahren an und für sich schneller ist als herkömmliche Techniken der Metallbearbeitung.

In einer anderen Ausführungsform des Marknagels weist die Zugfestigkeit des Schaftteils in radialer Richtung - von der Längsachse, oder von der Wand einer vorhandenen Kannullierung zur Oberfläche des Schaftteils hin - einen zunehmenden Gradienten auf. Damit ist der Vorteil erreichbar, dass während der Kaltverformung kein Dorn in die Kannulierung eingesetzt werden muss, so dass ein einfacheres Herstellungsverfahren erreichbar ist.

In wiederum einer anderen Ausführungsform weist die Zugfestigkeit des Schaftteils in radialer Richtung - von der äusseren Oberfläche des Schaftes zur Längsachse oder zur Wand einer Kannulierung hin - einen zuerst abnehmenden und dann wieder einen zunehmenden Gradienten aufweist. Gegenüber einem Verfahren ohne Einsatz eines Dornes in die Kannulierung während der Kaltverformung ist hier durch die Zunahme der Zugfestigkeit innen am Marknagel insgesamt eine höhere Materialfestigkeit erreichbar.

Je nach Ausführungsform des Marknagels:
- beträgt die axiale Länge des Anschlussteils höchstens 30 %, vorzugsweise höchstens 10 % der Gesamtlänge des Marknagels;
- weist die Oberfläche des Schaftes eine maximale Rauhigkeit Rₐ von 1,6 µm, vorzugsweise von maximal 0,8 µm auf;
- weist das Metall oder die Metallegierung des Schaftteils eine um mindestens 5 % höhere mechanische Festigkeit auf als das Metall oder die Metallegierung des Anschlussteils;
- weist das Metall oder die Metallegierung des Schaftteils eine höhere Zugfestigkeit auf als das Metall oder die Metallegierung des Anschlussteils;
- weist das Metall oder die Metallegierung des Schaftteils eine höhere Biegefestigkeit auf als das Metall oder die Metallegierung des Anschlussteils;
- weist das Metall oder die Metallegierung des Schaftteils eine höhere Torsionsfestigkeit auf als das Metall oder die Metallegierung des Anschlussteils;
- weist das Metall oder die Metallegierung des Schaftteils eine höhere Dauerwechselfestigkeit auf als das Metall oder die Metallegierung des Anschlussteils.

In einer weiteren Ausführungsform des Marknagels weisen der Anschlussteil und der Schaftteils materialmässig die gleiche Zusammensetzung auf, so dass der Marknagel einstückig herstellbar ist.

In wiederum einer weiteren Ausführungsform wird die höhere mechanische Festigkeit des Schaftteils durch Kaltverformung des Metalls oder der Metallegierung erzeugt. Der Vorteil besteht hier im wesentlichen in der einfachen Herstellung des Marknagels.

In einer anderen Ausführungsform ist der Aussendurchmesser D_{Rohr} des Anschlussteils grösser ist als der Aussendurchmesser D_{Schaft} des Schaftteils, so dass bei der Herstellung nur der Schaftteil kaltverformt werden muss.

In wiederum einer anderen Ausführungsform ist der Aussendurchmesser D_{Rohr} des Anschlussteils gleich gross ist wie der Aussendurchmesser D_{Schaft} des Schaftteils. Der Vorteil dieser Ausführungsform besteht in der im wesentlichen konstanten mechanischen Festigkeit des Marknagels über der gesamten Länge.

In einer weiteren Ausführungsform umfasst dieser eine zur Längsachse konzentrische Kannulierung, vorzugsweise in Form eines zylindrischen Hohlraums, wobei vorzugsweise die Wandstärke "W" des Anschlussteils grösser ist als die Wandstärke "w" des Schaftteils. Die Wandstärke w gehorcht vorzugsweise der Bedingung 0,60 W < w < 0,85 W.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch ein unbearbeitetes Rohr für eine erste Herstellungsvariante;
Fig. 2 einen Querschnitt längs der Linie II-II in Fig. 1 und Fig. 3;
Fig. 3 einen Längsschnitt durch einen erfindungsgemässen Marknagel mit einem verjüngten distalen Teil;
Fig. 4 einen Querschnitt längs der Linie III-III in Fig. 3;
Fig. 5 einen Längsschnitt durch ein unbearbeitetes Rohr für eine zweite Herstellungsvariante;
Fig. 6 einen Längsschnitt durch das teilweise bearbeitete Rohr nach Fig. 5 mit einem verjüngten Schaftteil;
Fig. 7 einen Längsschnitt durch das fertig bearbeitete Rohr nach Fig. 6 mit einem konstantem Aussendurchmesser als zweite Variante für einen erfindungsgemässen Marknagel;
Fig. 8 ein Diagramm der mech. Festigkeit des Schaftes eines erfindungsgemässen Marknagels; und
Fig. 9 ein Diagramm der mech. Festigkeit des Schaftes für eine Variante eines erfindungsgemässen Marknagels.

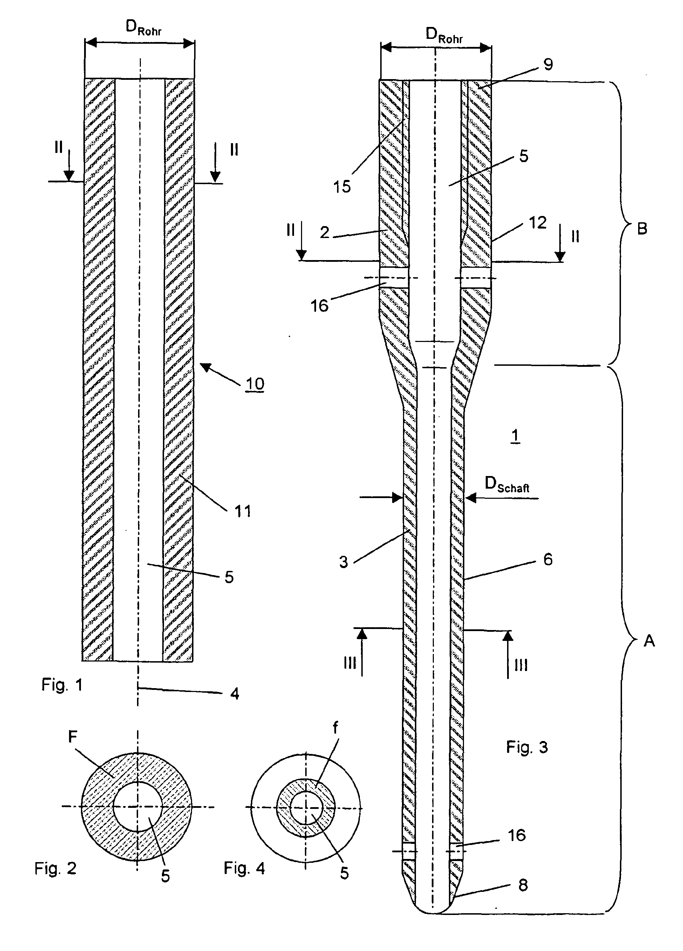

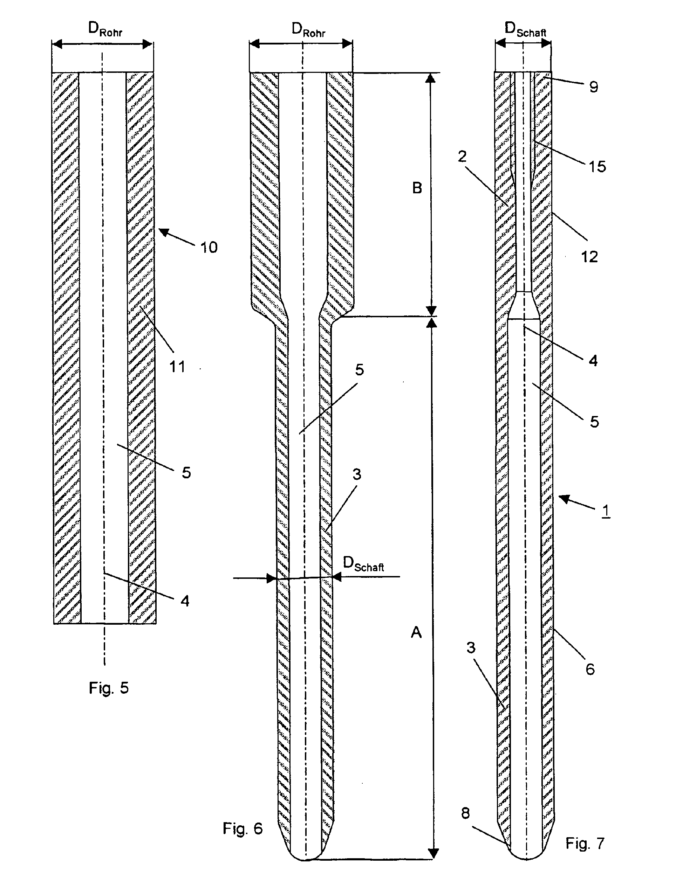

In den Fig. 1 und 5 ist ein unbearbeitetes hohlzylindrisches oder hohlprismatisches Rohr 10 mit einem Aussendurchmesser D_{Rohr} dargestellt, welches als Ausgangsstück für den Marknagel 1 dient. Das Rohr 10 weist eine zur Längsachse 4 koaxiale, von der Rohrwand 11 umschlossene Kannulierung 5 auf. Die zur Längsachse 4 orthogonale Querschnittsfläche F des unbearbeiteten Rohres 10 ist in Fig. 2 dargestellt.

Fig. 3 zeigt den Marknagel 1 nach seiner Kaltverformung. Nach der Kaltverformung ist der Marknagel 1 von seinem vorderen Ende 8 her auf einem den Schafteil 3 bildenden Abschnitt A seiner Länge gegenüber dem Rohr 10 als Ausgangsstück aussen diametral eingeschnürt und wahlweise, je nach Durchmesser des während der Verformung in der Kannulierung 5 eingesetzten Dornes auch innen im Durchmesser verringert oder unverändert. Die Oberfläche 6 des Schaftteils 3 mündet mit einem konusartigen Übergang in die Oberfläche 12 des Anschlussteils 2. Der kaltverformte Abschnitt A des Marknagels 1 weist eine zur Längsachse 4 orthogonale Querschnittsfläche f (Fig. 4) auf, welche kleiner als die Querschnittsfläche F ist. Der an das hintere Ende 9 des Marknagels 1 grenzende, unverformte Abschnitt B des Marknagels 1 bildet das Anschlussteil 2 des Marknagels 1 und weist den Aussendurchmesser D_{Rohr} des unverformten Rohres 10 (Fig. 1) auf. Im Anschlussteil 2 ist vom hinteren Ende 9 her ein Innengewinde 15 in die Kannulierung 5 geschnitten. Ferner sind am Anschlussteil 2 sowie am Schaftteil 3 je mindestens eine Querbohrung 16 mit quer zur Längsachse 4 stehender Bohrungsachse angeordnet, wobei der Winkel zwischen der Längsachse 4 und den Bohrungsachsen typischerweise zwischen 30° und 90° beträgt.

Fig. 6 zeigt einen aus dem Rohr 10 (Fig. 5) erzeugten Rohling mit einem Aussendurchmesser D_{Rohr} für eine andere Ausführungsform des Marknagels 1. Auch hier wurde das Rohr 10 (Fig.5) nur auf dem vom vorderen Ende 8 des Marknagels 1 her gemessenen, den Schaftteil 3 bildenden Abschnitt A der Länge bis zu einem Aussendurchmesser D_{Schaft} < D_{Rohr} eingeschnürt. Der vom hinteren Ende 9 her gemessene Abschnitt B der Länge des Marknagels 1 ist unverformt und weist weiterhin den Aussendurchmesser D_{Rohr} auf. Die Kannulierung 5 des Rohlinges ist im Abschnitt A wahlweise verengt oder unverändert, wobei die Ausgestaltung der Kannulierung 5 nach der Kaltverformung vom Durchmesser des während der Kaltverformung in der Kannulierung 5 eingesetzten Dornes abhängt.

Fig. 7 zeigt den in Fig. 6 dargestellten Rohling nach einer zweiten, nach der Verformung des Schafteiles 3 erfolgten Kaltverformung, welche nur auf dem das Anschlussteil 2 bildenden Abschnitt B erfolgte. Das Anschlussteil 2 (Abschnitt B) wurde so weit radial zusammengepresst bis sein Aussendurchmesser dem Aussendurchmesser D_{Schaft} des Schafteils 3 entspricht. Die Kannulierung 5 verjüngt sich beim Übergang vom Schaftteil 3 zum Anschlussteil 2 und weist hier im Anschlussteil 2 einen kleineren Durchmesser auf als im Schaftteil 5. Ferner ist vom hinteren Ende 9 her ein Innengewinde 15 in die Kannulierung 5 im Anschlussteil 2 geschnitten.

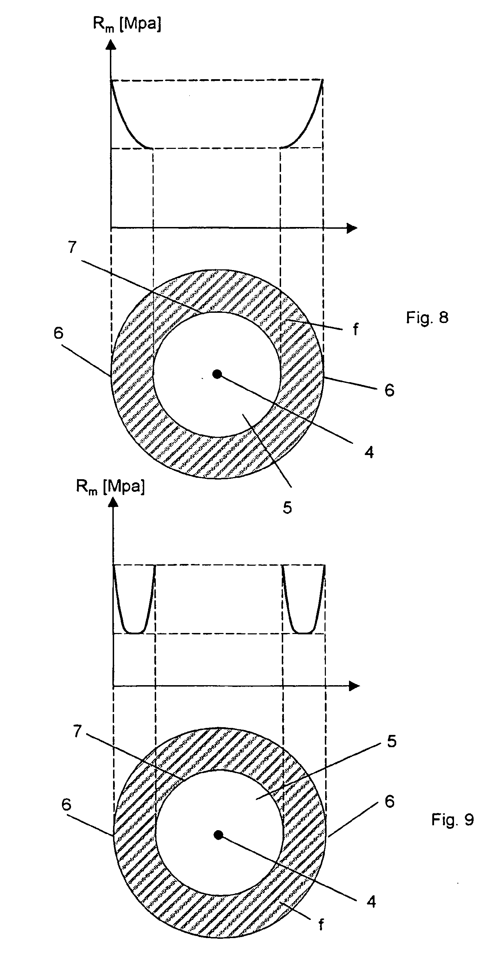

In Fig. 8 ist der Verlauf der Zugfestigkeit Rₘ in der Rohrwand 11 des kaltverformten Schaftteils 3 dargestellt. Die Zugfestigkeit Rₘ nimmt in diesem Fall in radialer Richtung von der Wand 7 der Kannulierung 5 zur Oberfläche 6 des Schaftteils 3 zu. Ein solcher Verlauf der Zugfestigkeit Rₘ in der Rohrwand 11 nach der Kaltverformung ist charakteristisch für eine Kaltverformung ohne Einsatz eines Dornes in die Kannulierung 5.

In Fig. 9 ist ein anderer Verlauf der Zugfestigkeit Rₘ nach abgeschlossener Kaltverformung dargestellt. Die Zugfestigkeit Rₘ weist in diesem Fall an der Wand 7 der Kannulierung 5 und an der Oberfläche 6 des Schaftteils 3 ein Maximum auf, während in der Mitte der Rohrwand 11 ein Minimum der Zugfestigkeit Rₘ vorliegt. Dieser Verlauf der Zugfestigkeit Rₘ in der Rohrwand 11 nach einer Kaltverformung ist charakteristisch für eine Kaltverformung mit Einsatz eines Dornes in die Kannulierung 5.

Im folgenden werden zwei verschiedene Herstellungsverfahren für den erfindungsgemässen Marknagel angegeben.

### Beispiel 1

Das vorliegende Beispiel entspricht den Figuren 1 bis 4.

Ein hohlzylindrisches oder hohlprismatisches Rohr 10 aus rostfreiem Stahl mit der Länge von typischerweise 100 bis 400 mm, einem Aussendurchmesser von typischerweise 10 bis 14 mm und einer Wandstärke zwischen 1,5 und 4,0 mm wird über einen ungefähr dem Schaftteil 3 des Marknagels 1 entsprechenden Abschnitt A von 70 bis 90 % der Rohrlänge aussen durch Kaltumformung bearbeitet, so dass sein Aussendurchmesser auf Werte zwischen 8 und 12 mm reduziert wird und damit das Rohr 10 um 20 bis 40 % verlängert wird, d.h. auf eine Endlänge von 120 bis 500 mm gebracht wird. Durch Einsetzen eines Dornes mit einem Aussendurchmesser von 5 mm bis 10 mm in die Kannulierung 5 des Rohres 10 während der Kaltverformung wird die Wandstärke des Rohres 10 auf 1 bis 3 mm reduziert.

Im Vergleich zu den Festigkeitswerten (Rₘ zwischen 500 und 800 MPa) des unbearbeiteten Rohres 10 weist das erfindungsgemäss bearbeitete Rohr 10 um 5 % bis 20 % höhere Festigkeitswerten (Rₘ Werte zwischen 600 und 1000 MPa) auf. Der derart erhaltene Rohling wird durch Anbringen von Querbohrungen 16 im Schaftteil 3 und im unbearbeiteten Rest des Rohres, d.h. im Anschlussteil 2 sowie eines koaxialen Innengewindes 15 in der Kannulierung 5 im Anschlussteil 2 zu einem Marknagel 1 verarbeitet.

### Beispiel 2

Das vorliegenden Beispiel entspricht den Figuren 5 bis 7.

Ein Rohr 10 aus rostfreiem Stahl mit der Länge von typischerweise 100 bis 400 mm, einem Aussendurchmesser von typischerweise 11 bis 17 mm und einer Wandstärke zwischen 1,5 und 4,0 mm wird über einen ungefähr dem Schaftteil 3 des Marknagels 1 entsprechenden Abschnitt A von 70 bis 90 % der Rohrlänge durch Kaltumformung bearbeitet, so dass sein Aussendurchmesser auf Werte zwischen 11 und 15 mm reduziert wird und damit das Rohr 10 um 20 bis 40 % verlängert wird, d.h. auf eine Endlänge von 120 bis 500 mm gebracht wird.

Dabei wird durch Einsetzen eines Dorns in das Innere des Rohres 10 auch seine Wandstärke auf Werte zwischen 1 bis 3 mm reduziert. Im Vergleich zu den Festigkeitswerten (Rₘ zwischen 500 und 800 MPa) des unbearbeiteten Rohres 10 weist das erfindungsgemäss bearbeitete Rohr 10 um 5 % bis 20 % höhere Festigkeitswerten (Rₘ Werte zwischen 600 und 1000 MPa) auf.

In einem weiteren Verfahrensschritt wird der Dorn im Inneren des Rohres 10 entfernt und das bisher unbearbeitete Anschlussteil 2 des Rohres 10 wird so umgeformt, dass das gesamte Rohr 10 einen konstanten Aussendurchmesser aufweist. In diesem Anschlussteil tritt eine geringere Erhöhung der Festigkeit auf, da sich das Material auf der Rohrinnenseite frei bewegen kann. Der derart erhaltene Rohling wird durch Anbringen von Querbohrungen 16 im Schaftteil 3 und Anschlussteil 2 sowie eines koaxialen Innengewindes 15 in der Kannulierung 5 des Anschlussteils 2 zu einem Marknagel 1 verarbeitet.

## Patentansprüche

1. Marknagel (1) aus einem Metall oder einer Metallegierung mit
A) einer Längsachse (4);
B) einem Anschlussteil (2), der ein zur Längsachse (4) orthogonales Querschnittsprofil F aufweist; und
C) einem zur Einführung in den Markkanal bestimmten Schaftteil (3), der ein zur Längsachse (4) orthogonales Querschnittsprofil f < F aufweist;
**dadurch gekennzeichnet, dass**
D) das Metall oder die Metallegierung des Schaftteils (3) eine höhere mechanische Festigkeit aufweist als das Metall oder die Metallegierung des Anschlussteils (2).

2. Marknagel (1) nach Anspruch 1, **dadurch gekennzeichnet, dass** die Zugfestigkeit Rₘ des Schaftteils (3) in radialer Richtung - von der Längsachse (4) zur Oberfläche (6) des Schaftteils (3) hin - einen zunehmenden Gradienten aufweist.

3. Marknagel (1) nach Anspruch 1, **dadurch gekennzeichnet, dass** die Zugfestigkeit Rₘ des Schaftteils (3) in radialer Richtung - von der Oberfläche (6) des Schaftes (3) zur Längsachse (4) hin - einen zuerst abnehmenden und dann wieder einen zunehmenden Gradienten aufweist.

4. Marknagel (1) nach einem der Ansprüche 1 bis 3, **dadurch gekennzeichnet, dass** die axiale Länge des Anschlussteils (2) höchstens 30 %, vorzugsweise höchstens 10 % der Gesamtlänge des Marknagels (1) beträgt.

5. Marknagel (1) nach einem der Ansprüche 1 bis 4, **dadurch gekennzeichnet, dass** die Oberfläche (6) des Schaftes (3) eine maximale Rauhigkeit Rₐ von 1,6 µm, vorzugsweise von maximal 0,8 µm aufweist.

6. Marknagel (1) nach einem der Ansprüche 1 bis 5, **dadurch gekennzeichnet, dass** das Metall oder die Metallegierung des Schaftteils (3) eine um mindestens 5 % höhere mechanische Festigkeit aufweist als das Metall oder die Metallegierung des Anschlussteils (2).

7. Marknagel (1) nach einem der Ansprüche 1 bis 6, **dadurch gekennzeichnet, dass** das Metall oder die Metallegierung des Schaftteils (3) eine höhere Zugfestigkeit aufweist als das Metall oder die Metallegierung des Anschlussteils (2).

8. Marknagel (1) nach einem der Ansprüche 1 bis 7, **dadurch gekennzeichnet, dass** das Metall oder die Metallegierung des Schaftteils (3) eine höhere Biegefestigkeit aufweist als das Metall oder die Metallegierung des Anschlussteils (2).

9. Marknagel (1) nach einem der Ansprüche 1 bis 8, **dadurch gekennzeichnet, dass** das Metall oder die Metallegierung des Schaftteils (3) eine höhere Torsionsfestigkeit aufweist als das Metall oder die Metallegierung des Anschlussteils (2).

10. Marknagel (1) nach einem der Ansprüche 1 bis 9, **dadurch gekennzeichnet, dass** das Metall oder die Metallegierung des Schaftteils (3) eine höhere Dauerwechselfestigkeit aufweist als das Metall oder die Metallegierung des Anschlussteils (2).

11. Marknagel (1) nach einem der Ansprüche 1 bis 10, **dadurch gekennzeichnet, dass** der Anschlussteil (2) und der Schaftteils (3) materialmässig die gleiche Zusammensetzung aufweisen.

12. Marknagel (1) nach einem der Ansprüche 1 bis 11, **dadurch gekennzeichnet, dass** die höhere mechanische Festigkeit des Schaftteils (3) durch Kaltverformung des Metalls oder der Metallegierung erzeugt ist.

13. Marknagel (1) nach einem der Ansprüche 1 bis 12, **dadurch gekennzeichnet, dass** der Aussendurchmesser D_{Rohr} des Anschlussteils (2) grösser ist als der Aussendurchmesser D_{Schaft} des Schaftteils (3).

14. Marknagel (1) nach einem der Ansprüche 1 bis 12, **dadurch gekennzeichnet, dass** der Aussendurchmesser D_{Rohr} des Anschlussteils (2) gleich gross ist wie der Aussendurchmesser D_{Schaft} des Schaftteils (3).

15. Marknagel (1) nach einem der Ansprüche 1 bis 14, **dadurch gekennzeichnet, dass** er eine zur Längsachse (4) konzentrische Kannulierung (5) aufweist, vorzugsweise in Form eines zylindrischen Hohlraums.

16. Marknagel (1) nach Anspruch 15, **dadurch gekennzeichnet, dass** die Wandstärke "W" des Anschlussteils (2) grösser ist als die Wandstärke "w" des Schaftteils (3).

17. Marknagel (1) nach Anspruch 16, **dadurch gekennzeichnet, dass** die Wandstärke w der Bedingung 0,60 W < w < 0,85 W gehorcht.

18. Verfahren zu Herstellung eines Marknagels (1) nach einem der Ansprüche 1 bis 17, **gekennzeichnet durch** Kaltverformung des zur Einführung in den Markkanal- bestimmten Schaftteils (3) eines Stabes oder Rohres mit konstanter Querschnittsfläche F, derart dass die Querschnittsfläche f des Schaftteils (3) zu f < F reduziert wird, und derart dass das Metall oder die Metallegierung des Schaftteils (3) eine höhere mechanische Festigkeit aufweist als das Metall oder die Metallegierung des Anschlussteils (2),

19. Verfahren nach Anspruch 18, **dadurch gekennzeichnet, dass** der Aussendurchmesser des Rohres konstant bleibt und durch die Kaltverformung eine Reduktion der Wandstärke im Schaftteil (3) resultiert.

20. Verfahren nach Anspruch 19, **dadurch gekennzeichnet, dass** durch die Kaltverformung die Wandstärke des Schaftteils (3) reduziert wird und gleichzeitig der Schaftteil (3) axial verlängert und in seinem Aussendurchmesser reduziert wird.

## Claims

1. Intramedullary nail (1) made of a metal or metal alloy, comprising
A) a longitudinal axis (4);
B) a connecting part (2) having a cross-sectional profile F orthogonal to the longitudinal axis (4) and
C) a shaft part (3) intended for insertion into the intramedullary canal, having a cross-sectional profile f < F orthogonal to the longitudinal axis (4),
**characterized in that**
D) the metal or the metal alloy of the shaft part (3) has a greater mechanical strength than the metal or the metal alloy of the connecting part (2).

2. Intramedullary nail (1) according to Claim 1, **characterized in that** the tensile strength Rₘ of the shaft part (3) has an increasing gradient in the radial direction-from the longitudinal axis (4) to the surface (6) of the shaft part (3).

3. Intramedullary nail (1) according to Claim 1, **characterized in that** the tensile strength Rₘ of the shaft part (3) has a gradient that first decreases and then increases again in the radial direction-from the surface (6) of the shaft part (3) toward the longitudinal axis (4).

4. Intramedullary nail (1) according to any one of Claims 1 through 3, **characterized in that** the axial length of the connecting part (2) is at most 30%, preferably at most 10% of the total length of the intramedullary nail (1).

5. Intramedullary nail (1) according to any one of Claims 1 through 4, **characterized in that** the surface (6) of the shaft (3) has a maximum roughness Rₐ of 1.6 µm, preferably max. 0.8 µm.

6. Intramedullary nail (1) according to any one of Claims 1 through 5, **characterized in that** the metal or metal alloy of the shaft part (3) has a mechanical strength at least 5% greater than that of the metal or metal alloy of the connecting part (2).

7. Intramedullary nail (1) according to any one of Claims 1 through 6, **characterized in that** the metal or metal alloy of the shaft part (3) has a higher tensile strength than the metal or metal alloy of the connecting part (2).

8. Intramedullary nail (1) according to any one of Claims 1 through 7, **characterized in that** the metal or metal alloy of the shaft part (3) has a higher bending strength than the metal or metal alloy of the connecting part (2).

9. Intramedullary nail (1) according to any one of Claims 1 through 8, **characterized in that** the metal or metal alloy of the shaft part (3) has a higher torsional strength than the metal or metal alloy of the connecting part (2).

10. Intramedullary nail (1) according to any one of Claims 1 through 9, **characterized in that** the metal or metal alloy of the shaft part (3) has a higher fatigue strength than the metal or metal alloy of the connecting part (2).

11. Intramedullary nail (1) according to any one of Claims 1 through 10, **characterized in that** the connecting part (2) and the shaft part (3) each have the same composition in terms of the material.

12. Intramedullary nail (1) according to any one of Claims 1 through 11, **characterized in that** the greater mechanical strength of the shaft part (3) is achieved by cold forming of the metal or metal alloy.

13. Intramedullary nail (1) according to any one of Claims 1 through 12, **characterized in that** the outside diameter D_{tube} of the connecting part (2) is greater than the outside diameter D_{shaft} of the shaft part (3).

14. Intramedullary nail (1) according to any one of Claims 1 through 12, **characterized in that** the outside diameter D_{tube} of the connecting part (2) the same as the outside diameter D_{shaft} of the shaft part (3).

15. Intramedullary nail (1) according to any one of Claims 1 through 14, **characterized in that** it has a cannulation (5) concentric with the longitudinal axis (4), preferably in the form of a cylindrical cavity.

16. Intramedullary nail (1) according to Claim 15, **characterized in that** the wall thickness "W" of the connecting part (2) is greater than the wall thickness "w" of the shaft part (3).

17. Intramedullary nail (1) according to Claim 16, **characterized in that** the wall thickness w conforms to the condition 0.60W < w < 0.85W.

18. Method for manufacturing an intramedullary nail (1) according to any one of Claims 1 through 17, **characterized by** cold forming of the shaft part (3) of a rod or a tube having a constant cross-sectional area F intended for insertion into the intramedullary canal such that the cross-sectional area F of the shaft part (3) is reduced to f < F and such that the metal or the metal alloy of the shaft part (3) has a greater mechanical strength than the metal or the metal alloy of the connecting part (2).

19. Method according to Claim 18, **characterized in that** the outside diameter of the tube remains constant and a reduction in wall thickness of the shaft part (3) is achieved by cold forming.

20. Method according to Claim 19, **characterized in that** the wall thickness of the shaft part (3) is reduced by the cold forming and at the same time the shaft part (3) is lengthened axially and its outside diameter is reduced.

## Revendications

1. Clou intramédullaire (1) fait de métal ou d'un alliage métallique, comprenant
A) un axe longitudinal (4) ;
B) une pièce de raccordement (2), qui présente un profil de section transversale F orthogonal à l'axe longitudinal (4) ; et
C) une partie de tige (3), conçue pour l'insertion dans le canal médullaire, qui présente un profil de section transversale f < F orthogonal à l'axe longitudinal (4) ;
**caractérisé en ce que**
D) le métal ou l'alliage métallique de la partie de tige (3) présente une résistance mécanique supérieure à celle du métal ou de l'alliage métallique de la pièce de raccordement (2).

2. Clou intramédullaire (1) selon la revendication 1, **caractérisé en ce que** la résistance à la traction Rₘ de la partie de tige (3) dans le sens radial - de l'axe longitudinal (4) à la surface (6) de la partie de tige (3) - présente un gradient croissant.

3. Clou intramédullaire (1) selon la revendication 1, **caractérisé en ce que** la résistance à la traction Rₘ de la partie de tige (3) dans le sens radial - de la surface (6) de la tige (3) à l'axe longitudinal (4) - présente un gradient d'abord décroissant puis de nouveau croissant.

4. Clou intramédullaire (1) selon l'une quelconque des revendications 1 à 3, **caractérisé en ce que** la longueur axiale de la pièce de raccordement (2) représente au maximum 30%, de préférence au maximum 10%, de la longueur totale du clou intramédullaire (1).

5. Clou intramédullaire (1) selon l'une quelconque des revendications 1 à 4, **caractérisé en ce que** la surface (6) de la tige (3) présente une rugosité maximale Rₐ de 1,6 µm, de préférence d'au maximum 0,8 µm.

6. Clou intramédullaire (1) selon l'une quelconque des revendications 1 à 5, **caractérisé en ce que** le métal ou l'alliage métallique de la partie de tige (3) présente une résistance mécanique supérieure d'au moins 5% à celle du métal ou de l'alliage métallique de la pièce de raccordement (2).

7. Clou intramédullaire (1) selon l'une quelconque des revendications 1 à 6, **caractérisé en ce que** le métal ou l'alliage métallique de la partie de tige (3) présente une résistance à la traction supérieure à celle du métal ou de l'alliage métallique de la pièce de raccordement (2).

8. Clou intramédullaire (1) selon l'une quelconque des revendications 1 à 7, **caractérisé en ce que** le métal ou l'alliage métallique de la partie de tige (3) présente une résistance à la flexion supérieure à celle du métal ou de l'alliage métallique de la pièce de raccordement (2).

9. Clou intramédullaire (1) selon l'une quelconque des revendications 1 à 8, **caractérisé en ce que** le métal ou l'alliage métallique de la partie de tige (3) présente une résistance à la torsion supérieure à celle du métal ou de l'alliage métallique de la pièce de raccordement (2).

10. Clou intraméduliaire (1) selon l'une quelconque des revendications 1 à 9, **caractérisé en ce que** le métal ou l'alliage métallique de la partie de tige (3) présente une résistance aux sollicitations alternées supérieure à celle du métal ou de l'alliage métallique de la pièce de raccordement (2).

11. Clou intramédullaire (1) selon l'une quelconque des revendications 1 à 10, **caractérisé en ce que** la pièce de raccordement (2) et la partie de tige (3) ont la même composition en termes de matériau.

12. Clou intramédullaire (1) selon l'une quelconque des revendications 1 à 11, **caractérisé en ce que** la résistance mécanique supérieure de la partie de tige (3) est obtenue par déformation à froid du métal ou de l'alliage métallique.

13. Clou intramédullaire (1) selon l'une quelconque des revendications 1 à 12, **caractérisé en ce que** le diamètre extérieur D_{tube} de la pièce de raccordement (2) est supérieur au diamètre extérieur D_{tige} de la partie de tige (3).

14. Clou intramédullaire (1) selon l'une quelconque des revendications 1 à 12, **caractérisé en ce que** le diamètre extérieur D_{tube} de la pièce de raccordement (2) est égal au diamètre extérieur D_{tige} de la partie de tige (3).

15. Clou intramédullaire (1) selon l'une quelconque des revendications 1 à 14, **caractérisé en ce qu'**il présente un canal (5) concentrique à l'axe longitudinal (4), de préférence sous la forme d'un espace creux cylindrique.

16. Clou intramédullaire (1) selon la revendication 15, **caractérisé en ce que** l'épaisseur de paroi "W" de la pièce de raccordement (2) est supérieure à l'épaisseur de paroi "w" de la partie de tige (3).

17. Clou intramédullaire (1) selon la revendication 16, **caractérisé en ce que** l'épaisseur de paroi w remplit la condition 0,60 W < w < 0,85 W.

18. Procédé de fabrication d'un clou intramédullaire (1) selon l'une quelconque des revendications 1 à 17, **caractérisé par** une déformation à froid de la partie de tige (3), conçue pour l'insertion dans le canal médullaire, d'une tige ou d'un tube à aire de section transversale constante F, de sorte que l'aire de section transversale f de la partie de tige (3) est réduite à f < F et de sorte que le métal ou l'alliage métallique de la partie de tige (3) présente une résistance mécanique supérieure à celle du métal ou de l'alliage métallique de la pièce de raccordement (2).

19. Procédé selon la revendication 18, **caractérisé en ce que** le diamètre extérieur du tube reste constant et que la déformation à froid entraîne une réduction de l'épaisseur de paroi dans la partie de tige (3).

20. Procédé selon la revendication 19, **caractérisé en ce que**, sous l'effet de la déformation à froid, l'épaisseur de paroi de la partie de tige (3) est réduite et, en même temps, la partie de tige (3) est allongée axialement et son diamètre extérieur est réduit.
